# EUROPEAN PATENT APPLICATION

(11) **EP 4 403 057 A1**
(43) Date of publication of application: **24.07.2024**
(21) Application number: 21968066.7
(22) Date of filing: 14.12.2021
(51) Int. Cl.: A24F 40/57, A24F 40/60, A24F 40/65

(54) **INFORMATION PROCESSING DEVICE, INFORMATION PROCESSING METHOD, AND PROGRAM**

(71) Applicant: Japan Tobacco, Inc., Tokyo, 105-6927 (JP)
(72) Inventor: INOUE, Yasunobu, Tokyo 130-8603 (JP); SUGANO, Yuka, Tokyo 130-8603 (JP); SENJU, Masatoshi, Tokyo 130-8603 (JP); SERITA, Kazutoshi, Tokyo 130-8603 (JP)
(74) Representative: Hoffmann Eitle
(86) International application number: PCT/JP2021/046060
(87) International publication number: WO 2023/112149

(57) **Abstract**

[Problem] To provide a mechanism which enables a user to more easily customize the operation of an inhalation device.

[Solution] This information processing device comprises a control unit that generates a display image displaying a profile, which is information representing a chronological change in a parameter related to an operation of generating aerosol performed by an inhalation device generating the aerosol using a base material, and changes the profile and display of the profile in the display image according to a user operation with respect to the profile in the generated display image. The control unit limits a range in which the profile can be changed, and changes the profile according to the user operation indicating a change to the profile within the range in which the profile can be changed.

## Description

### Technical Field

The present invention relates to an information processing device, an information processing method, and a program.

### Background Art

Inhaler devices, such as electronic cigarettes and nebulizers, which generate material to be inhaled by a user, are widespread. For example, by using a substrate including an aerosol source for generating an aerosol, a flavor source for imparting a flavor component to the generated aerosol, and the like, an inhaler device generates an aerosol imparted with the flavor component. The user can inhale the aerosol generated by the inhaler device and imparted with the flavor component to enjoy the flavor.

In recent years, various technologies related to inhaler devices have been developed to provide more enriched inhalation experiences to users. For example, Patent Literature 1 below discloses a technique for changing the temperature at which the inhaler device heats the substrate over time.

### Citation List

### Patent Literature

Patent Literature 1: JP 6125008 B2

### Summary of Invention

### Technical Problem

However, the technique disclosed in Patent Literature 1 above has not been developed to its full potential, and there has been a demand for further technical development for providing a more enriched inhalation experience to the user

Accordingly, the present invention has been made in view of the above problem, and an object of the present invention is to provide a mechanism that allows a user to more easily customize the operation of an inhaler device.

### Solution to Problem

In order to solve the above problem, according to an aspect of the present invention, there is provided an information processing device including: a controller configured to: generate a display image displaying a profile, the profile being information indicating a time-series change of a parameter related to an operation to be performed by an inhaler device that generates an aerosol by using a substrate, the operation being an operation of generating the aerosol; and change the profile and display of the profile in the display image in accordance with a user operation on the profile on the generated display image, in which the controller limits a range within which the profile is changeable, and changes the profile in accordance with the user operation for designating a change of the profile within the limited range within which the profile is changeable.

The controller may limit the range within which the profile is changeable, based on information on the inhaler device.

The inhaler device may include a heater configured to heat the substrate to generate the aerosol, and the information on the inhaler device may include at least one of an upper limit of a duration of a period during which the heater performs preliminary heating, an upper limit of a duration of a period during which the heater operates based on the profile, an upper limit of a temperature of the heater, an upper limit of a temperature rising rate of the heater, or an upper limit of a total energy amount to be consumed by the heater.

The controller may limit the range within which the profile is changeable, based on information on the substrate.

The information processing device may include a communicator configured to communicate with the inhaler device, and the controller may limit the range within which the profile is changeable, based on information received by the communicator.

The controller may change limitation on the range within which the profile is changeable, based on the user operation.

If the user operation for designating a change of the profile beyond the range within which the profile is changeable is performed, the controller may generate the display image displaying information indicating that the range within which the profile is changeable is exceeded.

If the user operation for designating a change of the profile beyond the range within which the profile is changeable is performed, the controller may generate the display image displaying information suggesting another change for enabling the change of the profile based on the user operation.

Limiting the range within which the profile is changeable may include at least one of limiting a range within which the parameter is changeable or limiting a period during which the parameter is changeable in the profile.

The inhaler device may include a heater configured to heat the substrate to generate the aerosol, and limiting the range within which the profile is changeable may include limiting a range within which a duration of a period during which the heater performs preliminary heating is changeable.

The inhaler device may include a heater configured to heat the substrate to generate the aerosol, and limiting the range within which the profile is changeable may include limiting a range within which a duration of a period during which the heater operates based on the profile is changeable.

The controller may generate the display image based on information on the substrate, the display image displaying information indicating whether the profile displayed on the display image is usable for the substrate.

The controller may generate the display image based on information on the substrate, the display image displaying information suggesting a change of the profile in a case where the profile displayed on the display image is to be used for the substrate.

The inhaler device may include a heater configured to heat the substrate to generate the aerosol, and the controller may generate the display image based on information on the substrate, the display image including information related to at least one of a duration of a period during which the aerosol is inhalable or a number of inhalable times in a case where the heater operates based on the profile.

The inhaler device may include a heater configured to heat the substrate to generate the aerosol, and changing the profile may include changing the parameter, changing a time corresponding to the parameter, and changing a duration of a period during which the heater operates based on the profile.

The inhaler device may include a heater configured to heat the substrate to generate the aerosol, and changing the profile may include changing a duration of a period during which the heater performs preliminary heating.

In order to solve the above problem, according to another aspect of the present invention, there is provided an information processing method including: generating a display image displaying a profile, the profile being information indicating a time-series change of a parameter related to an operation to be performed by an inhaler device that generates an aerosol by using a substrate, the operation being an operation of generating the aerosol; and limiting a range within which the profile is changeable and changing the profile and display of the profile on the display image in accordance with a user operation on the profile on the generated display image, the user operation being a user operation for designating a change of the profile within the limited range within which the profile is changeable.

In order to solve the above problem, according to another aspect of the present invention, there is provided a program for causing a computer to execute: generating a display image displaying a profile, the profile being information indicating a time-series change of a parameter related to an operation to be performed by an inhaler device that generates an aerosol by using a substrate, the operation being an operation of generating the aerosol; and limiting a range within which the profile is changeable and changing the profile and display of the profile on the display image in accordance with a user operation on the profile on the generated display image, the user operation being a user operation for designating a change of the profile within the limited range within which the profile is changeable.

### Advantageous Effects of Invention

As described above, according to the present invention, there is provided a mechanism that allows a user to more easily customize the operation of an inhaler device.

### Brief Description of Drawings

[Fig. 1] Fig. 1 is a schematic diagram schematically illustrating a configuration example of an inhaler device.
[Fig. 2] Fig. 2 is a diagram illustrating an example of a configuration of a system according to an embodiment of the present invention.
[Fig. 3] Fig. 3 is a graph illustrating an example of a transition of a temperature of a heater in a case where temperature control is performed based on a heating profile illustrated in Table 1.
[Fig. 4] Fig. 4 is a diagram illustrating an example of a display image generated by a terminal device according to the embodiment.
[Fig. 5] Fig. 5 is a diagram illustrating an example of the display image generated by the terminal device according to the embodiment.
[Fig. 6] Fig. 6 is a diagram illustrating an example of the display image generated by the terminal device according to the embodiment.
[Fig. 7] Fig. 7 is a diagram illustrating an example of the display image generated by the terminal device according to the embodiment.
[Fig. 8] Fig. 8 is a diagram illustrating an example of the display image generated by the terminal device according to the embodiment.
[Fig. 9] Fig. 9 is a diagram illustrating an example of the display image generated by the terminal device according to the embodiment.
[Fig. 10] Fig. 10 is a diagram illustrating an example of the display image generated by the terminal device according to the embodiment.
[Fig. 11] Fig. 11 is a diagram illustrating an example of the display image generated by the terminal device according to the embodiment.
[Fig. 12] Fig. 12 is a diagram illustrating an example of the display image generated by the terminal device according to the embodiment.
[Fig. 13] Fig. 13 is a diagram illustrating an example of the display image generated by the terminal device according to the embodiment.
[Fig. 14] Fig. 14 is a sequence diagram illustrating an example of a flow of a process executed by the system according to the embodiment.

### Description of Embodiments

A preferred embodiment of the present invention will be described below in detail with reference to the accompanying drawings. In the specification and the drawings, structural elements having substantially the same functional configuration are denoted by the same reference numerals, and redundant description thereof will be omitted.

### <1. Configuration example>

### <1.1. Configuration example of inhaler device>

An inhaler device generates material to be inhaled by a user. In the example described below, the material generated by the inhaler device is an aerosol. Alternatively, the material generated by the inhaler device may be gas.

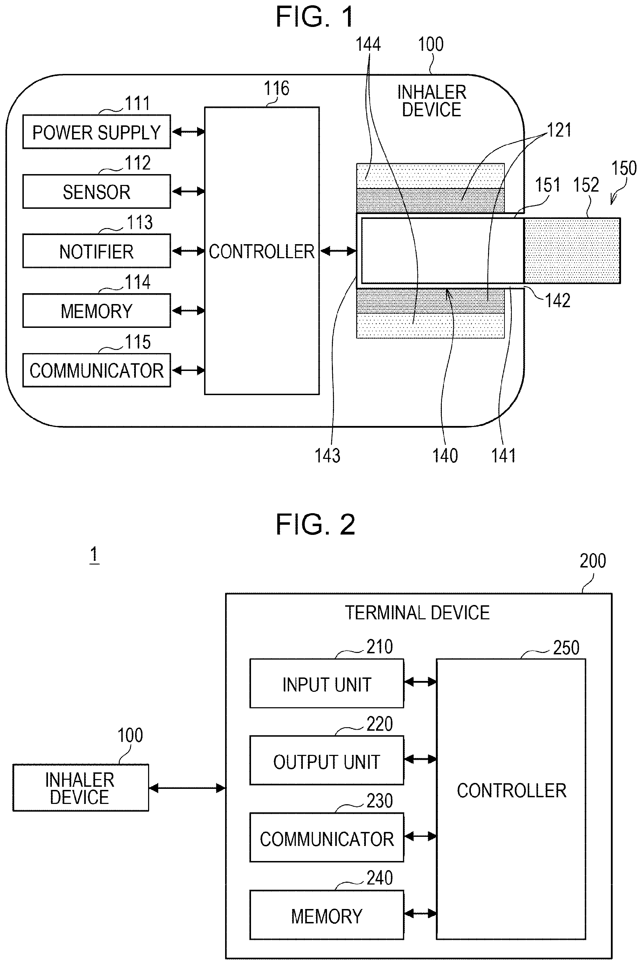

Fig. 1 is a schematic diagram schematically illustrating a configuration example of the inhaler device. As illustrated in Fig. 1, an inhaler device 100 according to the present configuration example includes a power supply 111, a sensor 112, a notifier 113, a memory 114, a communicator 115, a controller 116, a heater 121, a holder 140, and a heat insulator 144.

The power supply 111 stores electric power. The power supply 111 supplies electric power to the structural elements of the inhaler device 100 under the control of the controller 116. The power supply 111 may be a rechargeable battery such as a lithium ion secondary battery.

The sensor 112 acquires various items of information regarding the inhaler device 100. In an example, the sensor 112 is a pressure sensor such as a condenser microphone, a flow sensor, or a temperature sensor, and acquires a value generated in accordance with the user's inhalation. In another example, the sensor 112 is an input device that receives information input by the user, such as a button or a switch.

The notifier 113 notifies the user of information. The notifier 113 may be a light-emitting device that emits light, a display device that displays an image, a sound output device that outputs sound, or a vibration device that vibrates.

The memory 114 stores various items of information for the operation of the inhaler device 100. For example, the memory 114 is a non-volatile storage medium such as a flash memory.

The communicator 115 is a communication interface capable of communication in conformity with any wired or wireless communication standard. Such a communication standard may be, for example, Wi-Fi (registered trademark), Bluetooth (registered trademark), or a low power wide area (LPWA).

The controller 116 functions as an arithmetic processing unit and a control circuit, and controls the overall operation of the inhaler device 100 in accordance with various programs. The controller 116 is implemented by an electronic circuit such as a central processing unit (CPU) or a microprocessor, for example.

The holder 140 has an internal space 141, and holds a stick substrate 150 in a manner that the stick substrate 150 is partially accommodated in the internal space 141. The holder 140 has an opening 142 that allows the internal space 141 to communicate with outside. The holder 140 holds the stick substrate 150 inserted into the internal space 141 through the opening 142. For example, the holder 140 is a tubular body having the opening 142 and a bottom 143 on its ends, and may define the pillar-shaped internal space 141. The holder 140 connects with an airflow path that supplies air to the internal space 141. For example, a side surface of the inhaler device 100 has an air inlet hole, which is an inlet of air into the airflow path. For example, the bottom 143 has an air outlet hole, which is an outlet of the air from the airflow path to the internal space 141.

The stick substrate 150 includes a substrate 151 and an inhalation port 152. The substrate 151 includes an aerosol source. For example, the aerosol source may be a liquid, such as polyhydric alcohol or water. Examples of the polyhydric alcohol include glycerine and propylene glycol. The aerosol source may include a flavor component that is either derived from tobacco or not derived from tobacco. For the inhaler device 100 that is a medical inhaler such as a nebulizer, the aerosol source may include a medicine. In the present configuration example, the aerosol source is not limited to the liquid and may be a solid. In a state in which the stick substrate 150 is held by the holder 140, the substrate 151 is at least partially accommodated in the internal space 141, and the inhalation port 152 at least partially protrudes from the opening 142. When the user inhales with the inhalation port 152, protruding from the opening 142, in their mouth, air flows into the internal space 141 through the airflow path (not illustrated), and the air and an aerosol generated from the substrate 151 reach inside the mouth of the user.

The heater 121 heats the aerosol source to atomize the aerosol source and generate the aerosol. In the example illustrated in Fig. 1, the heater 121 has a film-like shape and surrounds the outer circumference of the holder 140. Subsequently, heat produced from the heater 121 heats the substrate 151 of the stick substrate 150 from the outer circumference, generating the aerosol. The heater 121 produces heat when receiving electric power from the power supply 111. In an example, the electric power may be supplied in response to the sensor 112 detecting a start of the user's inhalation and/or an input of predetermined information. Subsequently, the supply of the electric power may be stopped in response to the sensor 112 detecting an end of the user's inhalation and/or an input of predetermined information.

The heat insulator 144 prevents heat from transferring from the heater 121 to the other structural elements. For example, the heat insulator 144 may be a vacuum heat insulator or an aerogel heat insulator.

The configuration example of the inhaler device 100 has been described above. The inhaler device 100 is not limited to the above configuration, and may be configured in various ways as exemplified below.

In an example, the heater 121 may have a blade-like shape, and may be disposed so that the heater 121 protrudes from the bottom 143 of the holder 140 toward the internal space 141. In this case, the heater 121 having the blade-like shape is inserted into the substrate 151 of the stick substrate 150 and heats the substrate 151 of the stick substrate 150 from its inside. In another example, the heater 121 may be disposed so that the heater 121 covers the bottom 143 of the holder 140. In still another example, the heater 121 may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that partially opens and closes an outer shell defining the internal space 141. Examples of the opening/closing mechanism include a hinge. In addition, the holder 140 may accommodate the stick substrate 150 while sandwiching the stick substrate 150 inserted into the internal space 141 by opening and closing the outer shell. In this case, the heater 121 may be at the sandwiching position of the holder 140 and may produce heat while pressing the stick substrate 150.

In addition, means for atomizing the aerosol source is not limited to heating by the heater 121. For example, the means for atomizing the aerosol source may be induction heating. In this case, the inhaler device 100 includes at least an electromagnetic induction source such as a coil that generates a magnetic field instead of the heater 121. The inhaler device 100 may be provided with a susceptor that produces heat by the induction heating, or the stick substrate 150 may include the susceptor.

The stick substrate 150 is an example of the substrate that includes the aerosol source and that contributes to generation of the aerosol. In addition, by the combination of the inhaler device 100 and the stick substrate 150, the aerosol is generated. Therefore, the combination of the inhaler device 100 and the stick substrate 150 may be regarded as an aerosol generation system.

### <1.2. System configuration example>

Fig. 2 illustrates an example of the configuration of a system 1 according to an embodiment of the present invention. As illustrated in Fig. 2, the system 1 includes the inhaler device 100 and a terminal device 200. The inhaler device 100 has the configuration described above.

The terminal device 200 is a device used by the user of the inhaler device 100. The terminal device 200 is, for example, any information processing device such as a smartphone, a tablet terminal, or a wearable device. As illustrated in Fig. 2, the terminal device 200 includes an input unit 210, an output unit 220, a communicator 230, a memory 240, and a controller 250.

The input unit 210 has a function of receiving inputs of various items of information. The input unit 210 may include an input device that receives an input of information from the user. Examples of the input device include a button, a keyboard, a touch panel, and a microphone. The input unit 210 may further include various sensors such as an image sensor and an inertial sensor and may receive a user operation as an input.

The output unit 220 has a function of outputting information. The output unit 220 may include an output device that outputs information to the user. Examples of the output device include a display device that displays information, a light-emitting device that emits light, a vibration device that vibrates, and a sound output device that outputs sound. An example of the display device is a display. An example of the light-emitting device is a light emitting diode (LED). An example of the vibration device is an eccentric motor. An example of the sound output device is a speaker. The output unit 220 outputs information input from the controller 250 to notify the user of the information.

The communicator 230 is a communication interface for transmitting and receiving information between the terminal device 200 and other devices. The communicator 230 performs communication in conformity with any wired or wireless communication standard. Such a communication standard may be, for example, a wireless local area network (LAN), a wired LAN, Wi-Fi (registered trademark), or Bluetooth (registered trademark).

The memory 240 stores various items of information for the operation of the terminal device 200. The memory 240 may be a non-volatile storage medium such as a flash memory.

The controller 250 functions as an arithmetic processing unit or a control circuit, and controls the overall operation of the terminal device 200 in accordance with various programs. The controller 250 is implemented by an electronic circuit such as a central processing unit (CPU) or a microprocessor, for example. The controller 250 may further include a read only memory (ROM) that stores programs to be used, arithmetic parameters, and the like, and a random access memory (RAM) that temporarily stores parameters and the like that change as appropriate. The terminal device 200 executes various processes under the control of the controller 250. Examples of the processes controlled by the controller 250 include processing of information input by the input unit 210, output of information by the output unit 220, transmission and reception of information by the communicator 230, and storage and reading of information by the memory 240. The controller 250 also controls other processes executed by the terminal device 200, such as an input of information to each structural element and processes based on information output from each structural element.

Note that the functions of the controller 250 may be implemented by using an application. The application may be pre-installed or may be downloaded. Alternatively, the functions of the controller 250 may be implemented by progressive web apps (PWA).

### <2. Technical features>

### (1) Heating profile

The inhaler device 100 according to the present embodiment operates in accordance with a heating profile. The heating profile is information that defines an operation of generating an aerosol (i.e., an operation of heating the substrate), which is performed by the inhaler device 100. The inhaler device 100 heats the substrate in accordance with the heating profile to generate an aerosol.

More specifically, the heating profile is information indicating a time-series change of a parameter related to an operation of generating an aerosol, which is performed by the inhaler device 100. An example of the parameter is the temperature of the heater 121. The heating profile may alternatively be information in which a time-series transition of target values of the temperature of the heater 121 (hereinafter, also referred to as target temperatures) is defined. In this case, the controller 116 controls the operation of the heater 121 such that the temperature of the heater 121 (hereinafter, also referred to as actual temperature) transitions in the same manner as the target temperature defined in the heating profile. The heating profile is typically designed such that the user tastes an optimal flavor when inhaling the aerosol generated from the stick substrate 150. Thus, by controlling the operation of the heater 121 based on the heating profile, the flavor tasted by the user can be optimized.

The heating profile includes one or more combinations of the target temperature and information indicating a timing at which the target temperature is reached. The controller 116 controls the temperature of the heater 121 while switching the target temperature in accordance with the elapse of time from the start of heating based on the heating profile. Specifically, the controller 116 controls the temperature of the heater 121, based on the deviation between a current actual temperature and a target temperature corresponding to the elapsed time from the start of heating based on the heating profile. The temperature control of the heater 121 can be implemented by known feedback control, for example. The feedback control may be, for example, proportional-integral-differential controller (PID control). The controller 116 may cause electric power from the power supply 111 to be supplied to the heater 121 in the form of pulses based on pulse width modulation (PWM) or pulse frequency modulation (PFM). In this case, the controller 116 can adjust the duty ratio or frequency of electric power pulses in the feedback control to perform the temperature control of the heater 121. Alternatively, the controller 116 may perform simple on/off control in the feedback control. For example, until the actual temperature reaches the target temperature, the controller 116 may execute heating by the heater 121, and, once the actual temperature reaches the target temperature, the controller 116 may stop the heating by the heater 121, and then, if the actual temperature becomes lower than the target temperature, the controller 116 may execute the heating by the heater 121 again. The controller 116 may further adjust the voltage in the feedback control.

The temperature of the heater 121 can be quantified by, for example, measuring or estimating an electrical resistance value of the heater 121 (more precisely, a heating resistor included in the heater 121). This is because the electrical resistance value of the heating resistor changes with temperature. The electrical resistance value of the heating resistor can be estimated by, for example, measuring an amount of voltage drop across the heating resistor. The amount of voltage drop across the heating resistor can be measured by a voltage sensor that measures a potential difference to be applied to the heating resistor. In another example, the temperature of the heater 121 can be measured by a temperature sensor, such as a thermistor, installed near the heater 121.

A period from the start to the end of the process of generating the aerosol by using the stick substrate 150 may be hereinafter also preferred to as a heating session. In other words, the heating session is a period during which the operation of the heater 121 is controlled based on the heating profile. The start of the heating session is a timing at which heating based on the heating profile is started. The end of the heating session is a timing at which the amount of generated aerosol becomes insufficient.

The heating session includes a first-half preliminary heating period and a second-half puff allowed period. The puff allowed period is a period during which the aerosol can be inhaled. During the puff allowed period, a sufficient amount of aerosol is assumed to be generated. The preliminary heating period is a period from the start of heating to the start of the puff allowed period. The heating performed during the preliminary heating period is also referred to as preliminary heating. Here, the user is notified of the timing at which the puff allowed period starts (in other words, the timing at which the preliminary heating period ends). For example, at the start timing of which the puff allowed period, the notifier 113 outputs vibrations. With reference to the notification, the user can start to takes a puff. The preliminary heating period may be regarded as a period from the start of heating to the notification.

The user may further be notified of the timing at which the puff allowed period ends. The user may furthermore be notified of the timing that is predetermined time before the end of the puff allowed period (for example, the timing at which the supply of electric power to the heater 121 ends). In this case, with reference to the notification, the user can take a puff during the puff allowed period.

The heating profile may include a plurality of periods for which different target temperatures are set. The temperature control may be performed such that the target temperature that is set for a certain period is reached at a given timing in the period, or the temperature control may be performed such that the target temperature that is set for a certain period is reached at the end of the period. In any case, the temperature of the heater 121 can transition in the same manner as the transition of the target temperature defined in the heating profile.

Table 1 below illustrates an example of the heating profile.

**[Table 1]**

| Table 1. Example of heating profile | | |
|---|---|---|
| Period | Elapsed time from start of heating | Target temperature |
| Initial temperature rise period | 0 seconds to 17 seconds | 310°C |
| | 17 seconds to 35 seconds | 310°C |
| Intermediate temperature fall period | 35 seconds to 45 seconds | 260°C |
| Temperature re-rise period | 45 seconds to 180 seconds | 290°C |
| | 180 seconds to 260 seconds | 290°C |
| Heating end period | after 260 seconds | - |

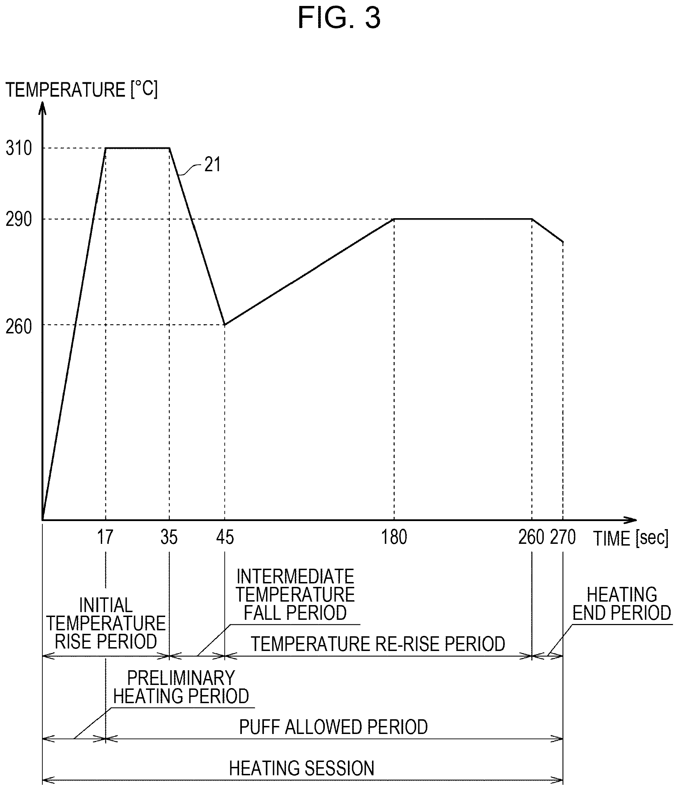

Now, referring to Fig. 3, the following will describe a transition of the temperature of the heater 121 in a case where the controller 116 performs the temperature control in accordance with the heating profile illustrated in Table 1. Fig. 3 is a graph illustrating an example of the transition of the temperature of the heater 121 in a case where the temperature control based on the heating profile illustrated in Table 1 is performed. In the graph, the horizontal axis represents time (sec). In the graph, the vertical axis represents the temperature of the heater 121. In the graph, a line 21 indicates the transition of the temperature of the heater 121. As illustrated in Fig. 3, the temperature of the heater 121 transitions in the same manner as the transition of the target temperature defined in the heating profile.

As illustrated in Table 1, the heating profile includes an initial temperature rise period at the beginning. The initial temperature rise period is a period during which the temperature of the heater 121 rises from an initial temperature. The initial temperature is the temperature of the heater 121 at the start of heating. As illustrated in Fig. 3, during the initial temperature rise period, the temperature of the heater 121 reaches 310°C at 17 seconds from the start of heating, and is maintained at 310°C until 35 seconds from the start of heating. Thus, the temperature of the stick substrate 150 is assumed to reach a temperature at which a sufficient amount of aerosol is generated. Since the temperature is increased to 310°C immediately at a time after the start of heating, the preliminary heating can be terminated in a short time, and the puff allowed period can be started in a short time. Note that the preliminary heating period ends at 17 seconds from the start of heating in Fig. 3.

As illustrated in Table 1, the heating profile includes an intermediate temperature fall period following the initial temperature rise period. The intermediate temperature fall period is a period during which the temperature of the heater 121 falls. As illustrated in Fig. 3, during the intermediate temperature fall period, the temperature of the heater 121 falls from 310°C to 260°C from 35 seconds to 45 seconds from the start of heating. During this period, the supply of electric power to the heater 121 may be stopped. Even in this case, by the remaining heat of the heater 121 and the stick substrate 150, a sufficient amount of aerosol is generated. Here, if the heater 121 is maintained at a high temperature, the aerosol source included in the stick substrate 150 is rapidly consumed, and the flavor tasted by the user may be degraded and may become, for example, excessively strong. In this regard, by providing the intermediate temperature fall period as an intermediate period, such degradation of the flavor can be avoided, and the user's puff experience quality can be enhanced.

As illustrated in Table 1, the heating profile includes a temperature re-rise period following the intermediate temperature fall period. The temperature re-rise period is a period during which the temperature of the heater 121 rises. As illustrated in Fig. 3, during the temperature re-rise period, the temperature of the heater 121 rises from 260°C to 290°C from 45 seconds to 180 seconds from the start of heating, and is maintained at 290°C until 260 seconds from the start of heating. If the temperature of the heater 121 is continuously allowed to fall, the temperature of the stick substrate 150 also falls, and the amount of generated aerosol is reduced, and the flavor tasted by the user may be degraded. In addition, as the heating profile advances toward the end, the remaining amount of the aerosol source included in the stick substrate 150 is reduced, and thus, even if the heating is continued at the same temperature, the amount of generated aerosol tends to be reduced. In this regard, by causing the temperature to rise again in the second half of the heating profile to increase the amount of generated aerosol, the reduction in the amount of generated aerosol due to the reduction in the remaining amount of the aerosol source can be compensated for Accordingly, even in the second half of the heating profile, it is possible to prevent the flavor tasted by the user from being degraded.

As illustrated in Table 1, the heating profile includes a heating end period at the end. The heating end period is a period following the temperature re-rise period, in which heating is not performed. No target temperature may be set. As illustrated in Fig. 3, the temperature of the heater 121 falls at and after 260 seconds from the start of heating. At 260 seconds from the start of heating, the supply of electric power to the heater 121 may end. Even in this case, by the remaining heat of the heater 121 and the stick substrate 150, a sufficient amount of aerosol is generated for a while. In the example illustrated in Fig. 3, at 270 seconds from the start of heating, the puff allowed period ends, that is, the heating session ends.

### (2) Customization of heating profile

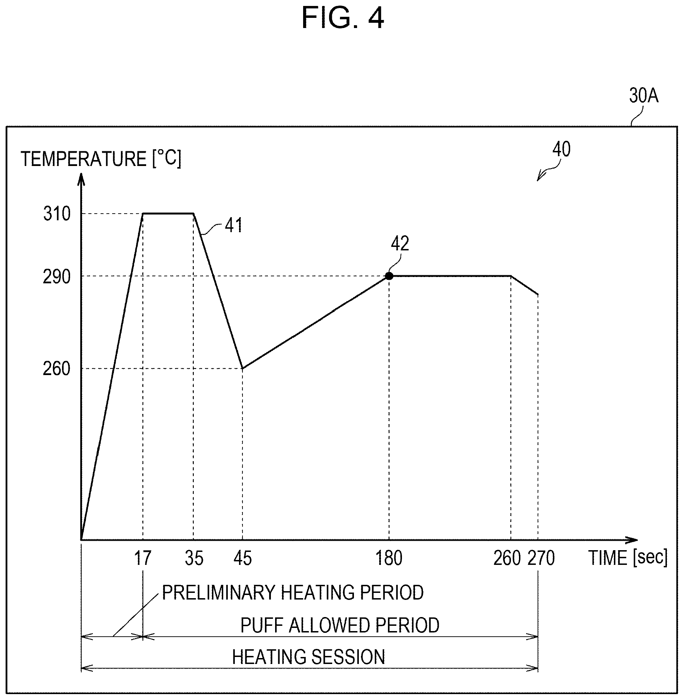

The terminal device 200 generates a display image displaying the heating profile. Then, the terminal device 200 changes the heating profile and the display of the heating profile in the display image in accordance with a user operation on the heating profile on the display image. This configuration allows the user to change the heating profile while visually recognizing the change of the heating profile. Accordingly, usability in customizing the heating profile is increased. Examples of the display image generated by the terminal device 200 will be described with reference to Figs. 4 to 8.

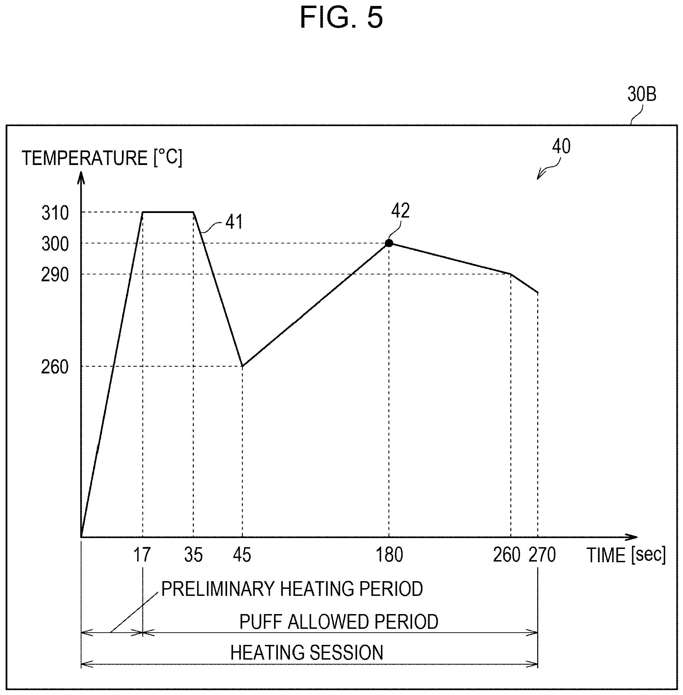

Figs. 4 to 8 are diagrams illustrating examples of the display image generated by the terminal device 200 according to the present embodiment. A display image 30A illustrated in Fig. 4 is a display image displaying an unchanged heating profile. Display images 30B to 30E illustrated in Figs. 5 to 8 are display images displaying heating profiles that are changed in accordance with user operations on the display image 30A illustrated in Fig. 4. The display images 30A to 30E include a graph 40 displaying a line 41 representing the heating profile. In the graph 40, the horizontal axis represents time. In the graph 40, the vertical axis represents the temperature of the heater 121 assumed if the heater 121 operates in accordance with the heating profile. The vertical axis of the graph 40 may simply be regarded as a parameter (that is, the target temperature of the heater 121) of the heating profile. The user can freely change the heating profile by operating a UI element displayed on the display image 30A.

Changing the heating profile may include changing the target temperature. For example, upon receiving a user operation for selecting a given point on the line 41 in the graph 40 and moving the point downward or upward, the terminal device 200 changes the target temperature corresponding to the point in accordance with the movement amount and the movement direction. Specifically, upon a user operation for moving a point 42 (180 seconds, 290°C) displayed on the display image 30A upward by 10°C, the terminal device 200 changes the heating profile such that the target temperature at 180 seconds from the start of heating is 300°C. Then, as illustrated in Fig. 5, the terminal device 200 generates and displays the display image 30B displaying the changed heating profile.

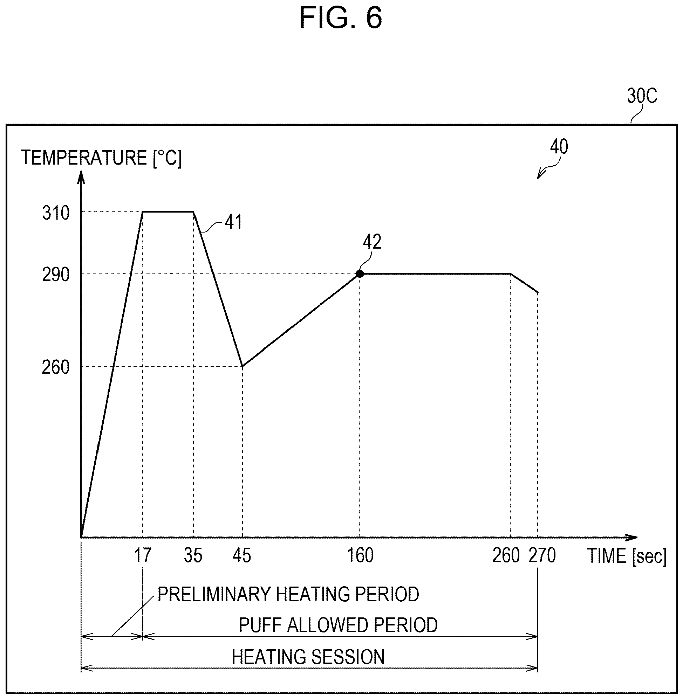

Changing the heating profile may include changing the time corresponding to the target temperature. For example, upon receiving a user operation for selecting a given point on the line 41 in the graph 40 and moving the point leftward or rightward, the terminal device 200 changes the time corresponding to the target temperature corresponding to the point in accordance with the movement amount and the movement direction. Specifically, upon a user operation for moving the point 42 (180 seconds, 290°C) displayed on the display image 30A leftward by 20 seconds, the terminal device 200 changes the heating profile such that the target temperature at 160 seconds from the start of heating is 290°C. Then, as illustrated in Fig. 6, the terminal device 200 generates and displays the display image 30C displaying the changed heating profile.

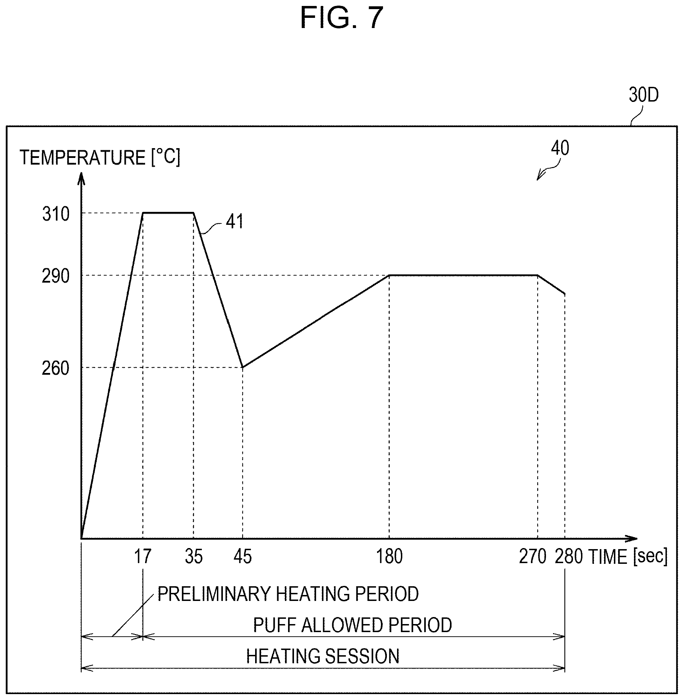

Changing the heating profile may include changing the duration of the heating session. For example, upon receiving a user operation for increasing or decreasing the width of the heating session displayed in the graph 40, the terminal device 200 changes the duration of the heating session in accordance with the increase/decrease direction and the increase/decrease amount. Specifically, upon a user operation for increasing the width of the heating session displayed on the display image 30A, which is 270 seconds, by 10 seconds, the terminal device 200 changes the heating profile such that the duration of the heating session is 280 seconds. Then, as illustrated in Fig. 7, the terminal device 200 generates and displays the display image 30D displaying the changed heating profile.

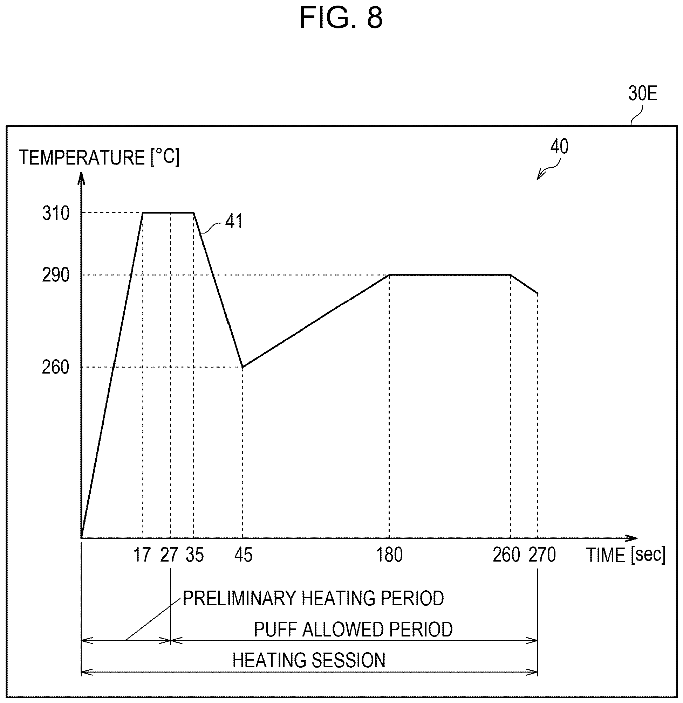

Changing the heating profile may include changing the duration of the preliminary heating period. For example, upon receiving a user operation for moving the position of the dividing line between the preliminary heating period and the puff allowed period displayed in the graph 40, the terminal device 200 changes the duration of the preliminary heating period in accordance with the movement amount and the movement direction. Specifically, upon a user operation for moving the position of the dividing line between the preliminary heating period and the puff allowed period displayed on the display image 30A, which is at 17 seconds from the start of heating, rightward by 10 seconds, the terminal device 200 changes the heating profile such that the duration of the preliminary heating period is 27 seconds. Then, as illustrated in Fig. 8, the terminal device 200 generates and displays the display image 30E displaying the changed heating profile.

Note that changing the duration of the preliminary heating period may be regarded as changing a timing for issuing a notification indicating the completion of preliminary heating.

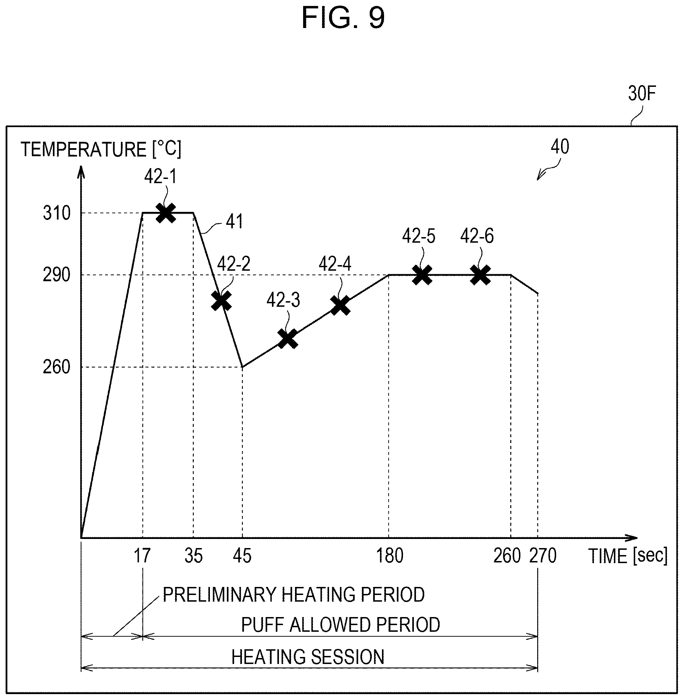

The display image may display the heating profile in association with information indicating a puff timing detected when the heating profile is previously used. In this case, the user can customize the heating profile while recognizing the relationship between the heating profile and the puff timing with reference to the feeling of inhaling the aerosol. An example of such a display image is illustrated in Fig. 9. Fig. 9 is a diagram illustrating an example of the display image generated by the terminal device 200 according to the present embodiment. A display image 30F illustrated in Fig. 9 includes the graph 40 displaying the line 41 representing the heating profile. In addition, X points 42 (42-1 to 42-6) on the line 41 in the graph 40 indicate points corresponding to puff timings detected when the heating profile is previously used, which is illustrated by the graph 40. The user can move these points 42 upward or downward to change the flavor at the puff timings.

The terminal device 200 controls the inhaler device 100 such that the inhaler device 100 operates in accordance with the changed heating profile. For example, the terminal device 200 receives the unchanged profile from the inhaler device 100, displays the unchanged profile, and transmits the heating profile changed by the user to the inhaler device 100. At this time, the terminal device 200 may transmit the changed heating profile or may transmit the difference between the unchanged heating profile and the changed heating profile. The next time an aerosol is generated, the inhaler device 100 operates in accordance with the changed heating profile. This configuration allows the user to freely customize the operation of the inhaler device 100. Accordingly, it becomes possible for the user to, for example, pursue a heating profile that achieves a desired smoking quality, with repeated customizations.

### (3) Limitation on customizable range

The terminal device 200 limits a heating profile changeable range. Then, in accordance with a user operation for designating a change of the heating profile within the limited heating profile changeable range, the terminal device 200 changes the profile. This configuration can prevent the user from unintentionally customizing the heating profile in an undesirable manner. That is, the user can customize the heating profile in accordance with the limitation by the terminal device 200 for easy customization as desired.

Limiting the heating profile changeable range may include limiting a range within which the target temperature is changeable. For example, the terminal device 200 may set at least one of an upper limit or a lower limit of the target temperature.

Limiting the heating profile changeable range may include limiting a period during which the target temperature is changeable in the heating profile. For example, the terminal device 200 may receive a change in the target temperature only within a certain period in the heating session.

Limiting the heating profile changeable range may include limiting a range within which the duration of the period during which the heater 121 performs the preliminary heating is changeable. For example, the terminal device 200 may set at least one of an upper limit or a lower limit of the duration of the preliminary heating period.

Limiting the heating profile changeable range may include limiting a range within which the duration of the period during which the heater 121 operates based on the heating profile is changeable. For example, the terminal device 200 may set at least one of an upper limit or a lower limit of the duration of the heating session.

The terminal device 200 may limit the heating profile changeable range, based on information on the inhaler device 100. The information on the inhaler device 100 may include information regarding hardware of the inhaler device 100, such as the characteristics of the power supply 111 and the characteristics of the heater 121. The information on the inhaler device 100 may also include information regarding software of the inhaler device 100, such as firmware defining the operation of the controller 116. This configuration allows customization of the heating profile within a range in which the inhaler device 100 is appropriately operable.

As an example, the information on the inhaler device 100 may include an upper limit of the duration of the preliminary heating period. In this case, the terminal device 200 sets the duration of the preliminary heating period in the heating profile to be changeable within the range lower than or equal to the upper limit. The inhaler device 100 may include a logic that determines that a malfunction has occurred if the temperature of the heater 121 does not reach a predetermined temperature within a predetermined time from the start of heating. If the preliminary heating is excessively long, this logic may cause an erroneous determination that a malfunction has occurred. In this regard, by setting the duration of the preliminary heating period to be changeable within the range lower than or equal to the upper limit, it is possible to prevent this logic from causing an erroneous determination that a malfunction has occurred. Furthermore, the information on the inhaler device 100 may also include a lower limit of the duration of the preliminary heating period.

As another example, the information on the inhaler device 100 may include an upper limit of the duration of the heating session. In this case, the terminal device 200 sets the duration of the heating session to be changeable within the range lower than or equal to the upper limit. If the heating session is excessively long, a malfunction may occur in the inhaler device 100 due to heat. In this regard, by setting the duration of the heating session to be changeable within the range lower than or equal to the upper limit, it is possible to prevent the occurrence of a malfunction in the inhaler device 100 due to heat. Furthermore, the information on the inhaler device 100 may also include a lower limit of the duration of the heating session.

As another example, the information on the inhaler device 100 may include an upper limit of the temperature of the heater 121 (that is, the target temperature). In this case, the terminal device 200 sets the target temperature defined in the heating profile to be changeable within the range lower than or equal to the upper limit. If the target temperature exceeds the heatproof temperature of the inhaler device 100, a malfunction may occur in the inhaler device 100 due to heat. In this regard, by setting the target temperature to be changeable within the range lower than or equal to the upper limit, it is possible to prevent the occurrence of a malfunction in the inhaler device 100 due to heat. Furthermore, the information on the inhaler device 100 may also include a lower limit of the temperature of the heater 121.

As an example, the information on the inhaler device 100 may include an upper limit of a rising rate of the temperature of the heater 121. In this case, the terminal device 200 sets a rising rate of the target temperature defined in the heating profile to be changeable within the range lower than or equal to the upper limit. Due to the voltage to be applied to the heater 121, the specifications of the heater 121, and the like, there is a limitation on the rising rate of the temperature of the heater 121. In this regard, by setting the rising rate of the target temperature to be changeable within the range lower than or equal to the upper limit, the temperature transition of the heater 121 in accordance with the heating profile can be achieved. Furthermore, the information on the inhaler device 100 may also include an upper limit of a falling rate of the temperature of the heater 121.

As an example, the information on the inhaler device 100 may include an upper limit of the total energy amount to be consumed by the heater 121. In this case, the terminal device 200 sets the heating profile to be changeable within the range lower than or equal to the upper limit of the total energy amount. The total energy amount corresponds to a value obtained by integrating the target temperature with the time regarding the time-series transition of the target temperature defined in the heating profile. In addition, the total energy amount corresponds to the amount of aerosol generated from the stick substrate 150. By setting the heating profile to be changeable within the range in which the total energy amount to be consumed by the heater 121 is lower than or equal to the upper limit, it is possible to prevent an excessive amount of aerosol from being generated. Furthermore, the information on the inhaler device 100 may also include a lower limit of the total energy amount to be consumed by the heater 121.

The terminal device 200 may limit the heating profile changeable range, based on information on the stick substrate 150. An example of the information on the stick substrate 150 is a type (hereinafter also referred to as brand) of the stick substrate 150. The terminal device 200 may limit the heating profile changeable range, based on the brand of the stick substrate 150 heated by the inhaler device 100. According to the brand of the stick substrate 150, the amounts and types of the included aerosol source and flavor component may differ, and the appropriate heating temperature and heating time may differ. In this regard, this configuration allows customization of the heating profile within the range in which the aerosol can be appropriately generated for each brand of the stick substrate 150.

The terminal device 200 may limit the heating profile changeable range, based on received information. For example, the terminal device 200 receives the information on the inhaler device 100 and/or the information on the stick substrate 150 from the inhaler device 100 and limits the heating profile changeable range, based on the received information, as described above. Note that the information on the stick substrate 150 may be identified by, for example, image recognition of the external appearance of the stick substrate 150 by the inhaler device 100. This configuration allows reduction of a load on the user to manually input the information on the inhaler device 100 and the information on the stick substrate 150. It is needless to say that the user may manually input the information on the inhaler device 100 and the information on the stick substrate 150 to the terminal device 200.

The terminal device 200 may change the limitation on the heating profile changeable range, based on a user operation. The user operation here is a user operation for changing the heating profile. For example, upon a user operation for increasing the target temperature in the first half of the heating profile, the terminal device 200 may decrease the upper limit of the target temperature in the second half of the heating profile as compared with that before the user operation. This configuration allows customization of the heating profile within the range in which the aerosol can be appropriately generated throughout the entire heating profile.

### (4) Customization guide

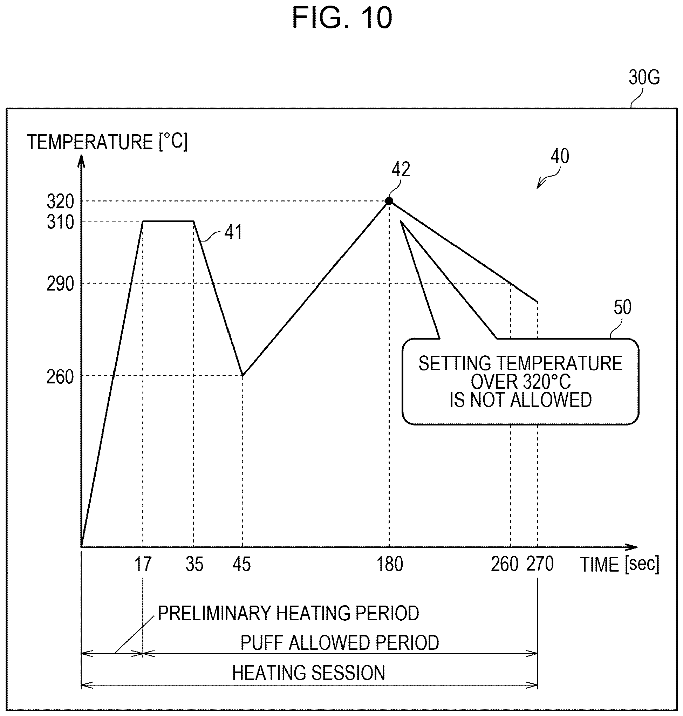

If a user operation for designating a change of the heating profile beyond the heating profile changeable range is performed, the terminal device 200 may generate a display image displaying information indicating that the heating profile changeable range is exceeded. More simply, if a user operation for designating a change of the heating profile beyond the heating profile changeable range is performed, the terminal device 200 may generate a display image displaying a warning. This configuration allows the user to change the heating profile within the heating profile changeable range. Accordingly, usability in customizing the heating profile is increased. An example of the display image generated by the terminal device 200 will be described with reference to Fig. 10.

Fig. 10 is a diagram illustrating an example of the display image generated by the terminal device 200 according to the present embodiment. A display image 30G illustrated in Fig. 10 is a display image displaying a heating profile that is changed in accordance with a user operation performed on the display image 30A illustrated in Fig. 4. The user operation is such that the point 42 (180 seconds, 290°C) displayed on the display image 30A is moved upward by 40°C. However, for example, if the upper limit of the temperature of the heater 121 is 320°C, the terminal device 200 limits the target temperature at 180 seconds from the start of heating to 320°C. Then, as illustrated in Fig. 10, the terminal device 200 generates and displays the display image 30G displaying a warning 50 indicating that it is not possible to set a temperature exceeding 320°C together with the graph 40 indicating the changed heating profile.

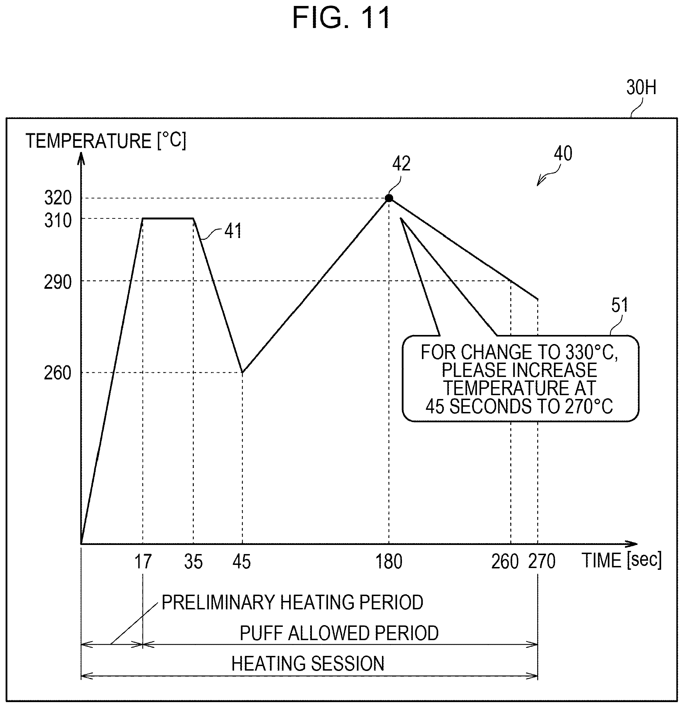

If a user operation for designating a change of the heating profile beyond the heating profile changeable range is performed, the terminal device 200 may generate a display image displaying information suggesting another change for enabling the change of the heating profile based on the user operation. As described above, the limitation on the heating profile changeable range may be changed based on a user operation. In this regard, if a user operation for designating another change is performed as suggested, the heating profile changeable range is changed, and the determination that the heating profile changeable range is exceeded may be overturned. This configuration allows the user to change the heating profile in accordance with the suggestion to achieve a desired change. Accordingly, usability in customizing the heating profile is increased. An example of the display image generated by the terminal device 200 will be described with reference to Fig. 11.

Fig. 11 is a diagram illustrating an example of the display image generated by the terminal device 200 according to the present embodiment. A display image 30H illustrated in Fig. 11 is a display image displaying a heating profile that is changed in accordance with a user operation performed on the display image 30A illustrated in Fig. 4. The user operation is such that the point 42 (180 seconds, 290°C) displayed on the display image 30A is moved upward by 40°C. However, if the upper limit to which the temperature can be increased in 135 seconds, which is from 45 seconds to 180 seconds from the start of heating, is 60°C, the temperature of the heater 121, which is 260°C at 45 seconds from the start of heating, can only be increased to 320°C at 180 seconds from the start of heating. Thus, the terminal device 200 limits the target temperature at 180 seconds from the start of heating to 320°C. Then, as illustrated in Fig. 11, the terminal device 200 displays the display image 30G displaying information 51 suggesting a change in the target temperature at 45 seconds from the start of heating to 270°C in order to change the target temperature at 180 seconds from the start of heating to 330°C. Once the user changes the target temperature at 45 seconds from the start of heating to 270°C in accordance with the information 51, it is possible to change the target temperature at 180 seconds from the start of heating to 330°C.

The brand of the stick substrate 150 used in the inhaler device 100 may be switched. In this case, the user is preferably notified of a relationship between the customized heating profile and the stick substrate 150 of the switched brand.

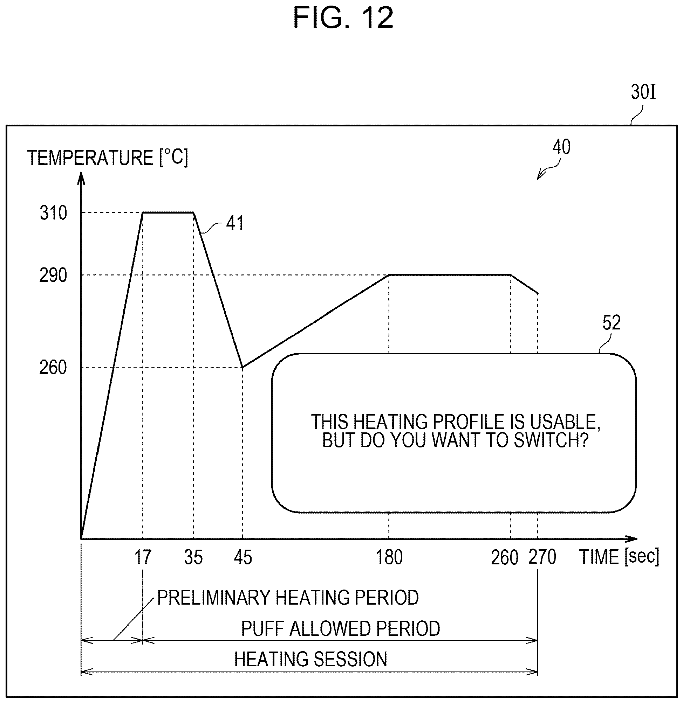

Thus, the terminal device 200 may generate, based on the information on the stick substrate 150, a display image displaying information indicating whether a heating profile displayed on the display image is usable for the stick substrate 150. For example, upon reception of information indicating the brand of the stick substrate 150 from the inhaler device 100, the terminal device 200 acquires information indicating the types and amounts of the aerosol source and the flavor source included in the stick substrate 150 of the brand indicated by the received information. These items of information may be stored in the memory 240 or may be acquired in response to an inquiry to a server or the like. Then, based on these items of information, the terminal device 200 generates a display image displaying information indicating whether the heating profile displayed on the display image is usable for the stick substrate 150. An example of the display image generated by the terminal device 200 will be described with reference to Fig. 12.

Fig. 12 is a diagram illustrating an example of the display image generated by the terminal device 200 according to the present embodiment. A display image 30I illustrated in Fig. 12 is an example of a display image displaying a heating profile and a display image displayed if the stick substrate 150 of a brand that is different from the previous one is inserted into the inhaler device 100. The display image 30I displays information 52 indicating that the heating profile illustrated by the graph 40 is also usable for the stick substrate 150 of the new brand. Thus, the user can select whether to use the heating profile without customization or with customization. Note that being also usable for the stick substrate 150 of the new brand means that the heating profile indicated by the graph 40 is within the heating profile changeable range limited based on the information on the stick substrate 150.

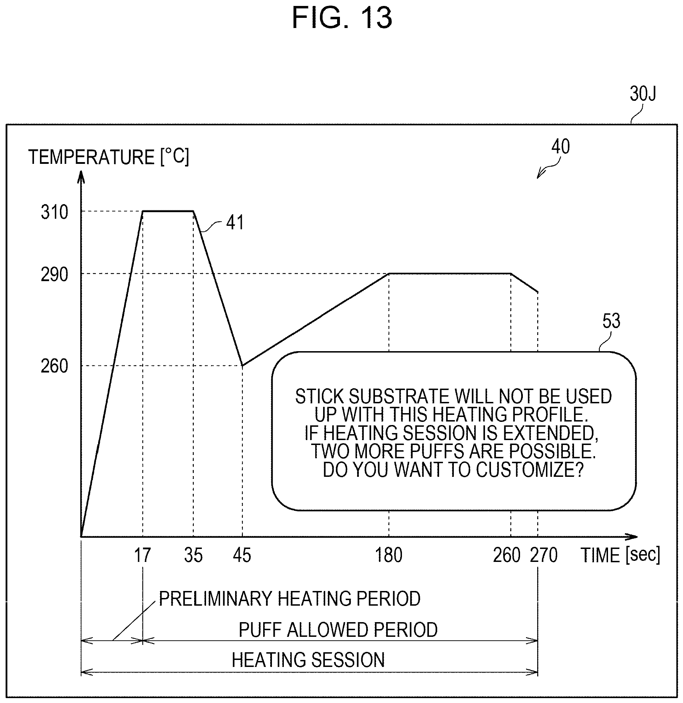

Even if the heating profile is usable for the stick substrate 150 of the new brand, preferable customization is desirably suggested to the user. Thus, in a case where the heating profile displayed on the display image is to be used for the stick substrate 150, the terminal device 200 may generate, based on the information on the stick substrate 150, a display image displaying information suggesting a change of the heating profile. Furthermore, in a case where the heater 121 operates based on the heating profile, the terminal device 200 may generate, based on the information on the stick substrate 150, a display image including information regarding at least one of the duration of the puff allowed period or an allowed number of puffs. For example, if the heating profile is changed, a display image including information indicating an increase in the duration of the puff allowed period or an increase in the allowed number of puffs may be generated. This configuration allows the user to attempt to change the heating profile for achieving an enriched user experience by using the stick substrate 150 of the new brand. An example of the display image generated by the terminal device 200 will be described with reference to Fig. 13.

Fig. 13 is a diagram illustrating an example of the display image generated by the terminal device 200 according to the present embodiment. A display image 30J illustrated in Fig. 13 is an example of a display image displaying a heating profile and a display image displayed if the stick substrate 150 of a brand that is different from the previous one is inserted into the inhaler device 100. The display image 30J indicates that the heating profile indicated by the graph 40 is also usable for the stick substrate 150 of the new brand and displays information 53 suggesting a change of the heating profile. Specifically, the information 53 suggests an increase in the duration of the heating session and indicates that two more puffs can be taken if the duration of the heating session is increased. Accordingly, by customizing the heating profile in accordance with this suggestion, the user can increase the number of puffs.

### (5) Process flow

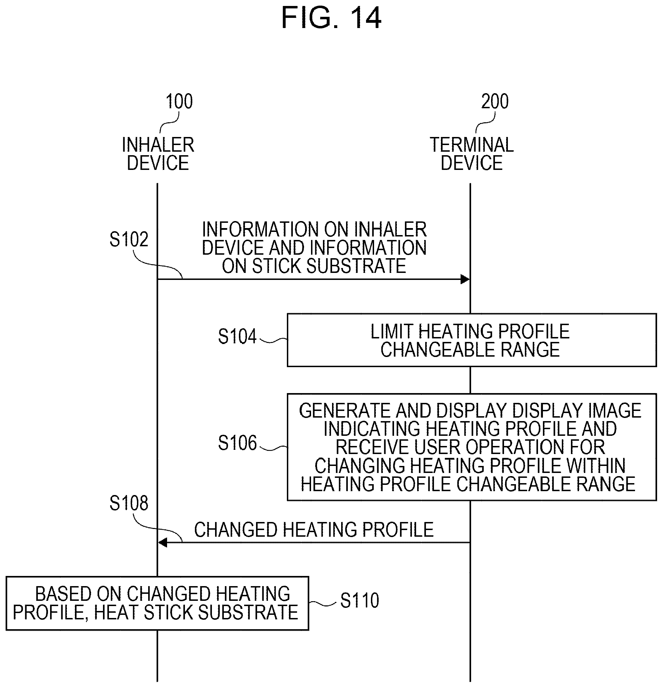

A process flow according to the present embodiment will be described below with reference to Fig. 14. Fig. 14 is a sequence diagram illustrating an example of a flow of a process executed by the system 1 according to the present embodiment. This sequence involves the inhaler device 100 and the terminal device 200.

As illustrated in Fig. 14, first, the inhaler device 100 transmits, to the terminal device 200, information on the inhaler device 100 and information on the stick substrate 150 (step S102). For example, the inhaler device 100 transmits information regarding hardware or software of the inhaler device 100. In addition, the inhaler device 100 transmits, to the terminal device 200, information indicating the brand of the stick substrate 150 held by the holder 140.

Subsequently, the terminal device 200 limits the heating profile changeable range (step S104). For example, based on the information received in step S102, the terminal device 200 sets an upper limit of the duration of the heating period, an upper limit of the duration of the heating session, an upper limit of the target temperature, and an upper limit of the total energy amount to be consumed by the heater 121.

Subsequently, the terminal device 200 generates and displays a display image indicating the heating profile, and receives a user operation for changing the heating profile within the heating profile changeable range (step S106). Based on the user operation performed within the heating profile changeable range, the terminal device 200 changes the heating profile. In addition, upon a user operation for designating a change of the heating profile beyond the heating profile changeable range, the terminal device 200 displays a warning or information suggesting another change.

Subsequently, the terminal device 200 transmits, to the inhaler device 100, information indicating the changed heating profile (step S108).

Subsequently, based on the changed heating profile indicated by the received information, the inhaler device 100 heats the stick substrate 150 held by the holder 140 (step S110).

### <3. Supplementary note>

While a preferred embodiment of the present invention has been described above in detail with reference to the accompanying drawings, the present invention is not limited to this example. It will be apparent that those skilled in the art to which the present invention belongs can achieve various modifications or variations without departing from the scope of the technical concept presented in the claims, and it is understood that such modifications or variations also fall within the technical scope of the present invention.

For example, in the embodiment described above, an example has been described in which the parameter for the heating profile is the temperature of the heater 121. However, the present invention is not limited to this example. In an example, the parameter may be a temperature of a portion heated by the heater 121. Examples of the portion heated by the heater 121 include the holder 140. In this case, the controller 116 controls the supply of electric power to the heater 121 such that the same temperature as a target temperature defined in the heating profile is achieved in the holder 140. In another example, the parameter may be information related to electricity to be supplied to the heater 121. For example, the parameter may be a voltage, a current, a resistance, or an electric power to be supplied to the heater 121. In this case, the controller 116 controls the supply of electric power to the heater 121 such that the same voltage, current, resistance, or electric power as the voltage, current, resistance, or electric power defined in the heating profile is supplied to the heater 121.

For example, the above embodiment has described an example in which the inhaler device 100 is configured as a device that heats a substrate including an aerosol source to generate an aerosol. However, the present invention is not limited to this example. The inhaler device 100 may also be configured as a so-called liquid atomization device, which heats a liquid guided from a substrate including an aerosol source as a liquid to generate an aerosol. In the liquid atomization device, upon detection of a puff, heating is performed to generate the aerosol. Therefore, a profile in the liquid atomization device is a file defining a temperature of heating performed upon detection of a puff. The present invention is also applicable to customization of such a profile.

The devices described herein may be implemented as independent devices, and all or some of them may be implemented as separate devices. For example, in the functional configuration example of the terminal device 200 illustrated in Fig. 3, the function of generating a display image and the function of changing a heating profile in accordance with a user operation, which are included in the controller 250, may be provided for a device such as a server connected to the terminal device 200 via a network or the like. Furthermore, the function of generating a display image and the function of changing a heating profile in accordance with a user operation, which are included in the controller 250, may be provided for the inhaler device 100.

A series of processes performed by each of the devices described herein may be implemented using any one of software, hardware, and a combination of software and hardware. A program constituting the software is stored in advance in, for example, an internal or external recording medium (non-transitory media) of each device. For example, each program is read into a RAM at the time of execution by a computer and executed by a processor such as a CPU. Examples of the recording media include a magnetic disk, an optical disk, a magneto-optical disk, and a flash memory. Furthermore, the computer program described above may be distributed via, for example, a network without using a recording medium.

The processes described herein using the flowchart and the sequence diagram may not necessarily be executed in the illustrated order. Some processing steps may be executed in parallel. Any additional processing step may be used, or some processing steps may be skipped.

The following configurations also fall within the technical scope of the present invention.
(1) An information processing device including:
   a controller configured to:
   generate a display image displaying a profile, the profile being information indicating a time-series change of a parameter related to an operation to be performed by an inhaler device that generates an aerosol by using a substrate, the operation being an operation of generating the aerosol; and
   change the profile and display of the profile in the display image in accordance with a user operation on the profile on the generated display image, in which
   the controller limits a range within which the profile is changeable, and changes the profile in accordance with the user operation for designating a change of the profile within the limited range within which the profile is changeable.
(2) The information processing device according to (1), in which
   the controller limits the range within which the profile is changeable, based on information on the inhaler device.
(3) The information processing device according to (2), in which
   the inhaler device includes a heater configured to heat the substrate to generate the aerosol, and
   the information on the inhaler device includes at least one of an upper limit of a duration of a period during which the heater performs preliminary heating, an upper limit of a duration of a period during which the heater operates based on the profile, an upper limit of a temperature of the heater, an upper limit of a temperature rising rate of the heater, or an upper limit of a total energy amount to be consumed by the heater.
(4) The information processing device according to any one of (1) to (3), in which
   the controller limits the range within which the profile is changeable, based on information on the substrate.
(5) The information processing device according to any one of (2) to (4), including
   a communicator configured to communicate with the inhaler device, in which
   the controller limits the range within which the profile is changeable, based on information received by the communicator.
(6) The information processing device according to any one of (1) to (5), in which
   the controller changes limitation on the range within which the profile is changeable, based on the user operation.
(7) The information processing device according to any one of (1) to (6), in which
   if the user operation for designating a change of the profile beyond the range within which the profile is changeable is performed, the controller generates the display image displaying information indicating that the range within which the profile is changeable is exceeded.
(8) The information processing device according to any one of (1) to (7), in which
   if the user operation for designating a change of the profile beyond the range within which the profile is changeable is performed, the controller generates the display image displaying information suggesting another change for enabling the change of the profile based on the user operation.
(9) The information processing device according to any one of (1) to (8), in which
   limiting the range within which the profile is changeable includes at least one of limiting a range within which the parameter is changeable or limiting a period during which the parameter is changeable in the profile.
(10) The information processing device according to any one of (1) to (9), in which
   the inhaler device includes a heater configured to heat the substrate to generate the aerosol, and
   limiting the range within which the profile is changeable includes limiting a range within which a duration of a period during which the heater performs preliminary heating is changeable.
(11) The information processing device according to any one of (1) to (10), in which
   the inhaler device includes a heater configured to heat the substrate to generate the aerosol, and
   limiting the range within which the profile is changeable includes limiting a range within which a duration of a period during which the heater operates based on the profile is changeable.
(12) The information processing device according to any one of (1) to (11), in which
   the controller generates the display image based on information on the substrate, the display image displaying information indicating whether the profile displayed on the display image is usable for the substrate.
(13) The information processing device according to any one of (1) to (12), in which
   the controller generates the display image based on information on the substrate, the display image displaying information suggesting a change of the profile in a case where the profile displayed on the display image is to be used for the substrate.
(14) The information processing device according to any one of (1) to (13), in which
   the inhaler device includes a heater configured to heat the substrate to generate the aerosol, and
   the controller generates the display image based on information on the substrate, the display image including information related to at least one of a duration of a period during which the aerosol is inhalable or a number of inhalable times in a case where the heater operates based on the profile.
(15) The information processing device according to any one of (1) to (14), in which
   the inhaler device includes a heater configured to heat the substrate to generate the aerosol, and
   changing the profile includes changing the parameter, changing a time corresponding to the parameter, and changing a duration of a period during which the heater operates based on the profile.
(16) The information processing device according to any one of (1) to (15), in which
   the inhaler device includes a heater configured to heat the substrate to generate the aerosol, and
   changing the profile includes changing a duration of a period during which the heater performs preliminary heating.
(17) An information processing method including:
   generating a display image displaying a profile, the profile being information indicating a time-series change of a parameter related to an operation to be performed by an inhaler device that generates an aerosol by using a substrate, the operation being an operation of generating the aerosol; and
   limiting a range within which the profile is changeable and changing the profile and display of the profile on the display image in accordance with a user operation on the profile on the generated display image, the user operation being a user operation for designating a change of the profile within the limited range within which the profile is changeable.
(18) A program for causing a computer to execute:
   generating a display image displaying a profile, the profile being information indicating a time-series change of a parameter related to an operation to be performed by an inhaler device that generates an aerosol by using a substrate, the operation being an operation of generating the aerosol; and
   limiting a range within which the profile is changeable and changing the profile and display of the profile on the display image in accordance with a user operation on the profile on the generated display image, the user operation being a user operation for designating a change of the profile within the limited range within which the profile is changeable.

### Reference Signs List

- 1: system
- 100: inhaler device
- 111: power supply
- 112: sensor
- 113: notifier
- 114: memory
- 115: communicator
- 116: controller
- 121: heater
- 140: holder
- 141: internal space
- 142: opening
- 143: bottom
- 144: heat insulator
- 150: stick substrate
- 151: substrate
- 152: inhalation port
- 200: terminal device
- 210: input unit
- 220: output unit
- 230: communicator
- 240: memory
- 250: controller

## Claims

1. An information processing device comprising:
a controller configured to:
generate a display image displaying a profile, the profile being information indicating a time-series change of a parameter related to an operation to be performed by an inhaler device that generates an aerosol by using a substrate, the operation being an operation of generating the aerosol; and
change the profile and display of the profile in the display image in accordance with a user operation on the profile on the generated display image, wherein
the controller limits a range within which the profile is changeable, and changes the profile in accordance with the user operation for designating a change of the profile within the limited range within which the profile is changeable.

2. The information processing device according to claim 1, wherein
the controller limits the range within which the profile is changeable, based on information on the inhaler device.

3. The information processing device according to claim 2, wherein
the inhaler device comprises a heater configured to heat the substrate to generate the aerosol, and
the information on the inhaler device comprises at least one of an upper limit of a duration of a period during which the heater performs preliminary heating, an upper limit of a duration of a period during which the heater operates based on the profile, an upper limit of a temperature of the heater, an upper limit of a temperature rising rate of the heater, or an upper limit of a total energy amount to be consumed by the heater.

4. The information processing device according to any one of claims 1 to 3, wherein
the controller limits the range within which the profile is changeable, based on information on the substrate.

5. The information processing device according to any one of claims 2 to 4, comprising
a communicator configured to communicate with the inhaler device, wherein
the controller limits the range within which the profile is changeable, based on information received by the communicator.

6. The information processing device according to any one of claims 1 to 5, wherein
the controller changes limitation on the range within which the profile is changeable, based on the user operation.

7. The information processing device according to any one of claims 1 to 6, wherein
if the user operation for designating a change of the profile beyond the range within which the profile is changeable is performed, the controller generates the display image displaying information indicating that the range within which the profile is changeable is exceeded.

8. The information processing device according to any one of claims 1 to 7, wherein
if the user operation for designating a change of the profile beyond the range within which the profile is changeable is performed, the controller generates the display image displaying information suggesting another change for enabling the change of the profile based on the user operation.

9. The information processing device according to any one of claims 1 to 8, wherein
limiting the range within which the profile is changeable comprises at least one of limiting a range within which the parameter is changeable or limiting a period during which the parameter is changeable in the profile.

10. The information processing device according to any one of claims 1 to 9, wherein
the inhaler device comprises a heater configured to heat the substrate to generate the aerosol, and
limiting the range within which the profile is changeable comprises limiting a range within which a duration of a period during which the heater performs preliminary heating is changeable.

11. The information processing device according to any one of claims 1 to 10, wherein
the inhaler device comprises a heater configured to heat the substrate to generate the aerosol, and
limiting the range within which the profile is changeable comprises limiting a range within which a duration of a period during which the heater operates based on the profile is changeable.

12. The information processing device according to any one of claims 1 to 11, wherein
the controller generates the display image based on information on the substrate, the display image displaying information indicating whether the profile displayed on the display image is usable for the substrate.

13. The information processing device according to any one of claims 1 to 12, wherein
the controller generates the display image based on information on the substrate, the display image displaying information suggesting a change of the profile in a case where the profile displayed on the display image is to be used for the substrate.

14. The information processing device according to any one of claims 1 to 13, wherein
the inhaler device comprises a heater configured to heat the substrate to generate the aerosol, and
the controller generates the display image based on information on the substrate, the display image comprising information related to at least one of a duration of a period during which the aerosol is inhalable or a number of inhalable times in a case where the heater operates based on the profile.

15. The information processing device according to any one of claims 1 to 14, wherein
the inhaler device comprises a heater configured to heat the substrate to generate the aerosol, and
changing the profile comprises changing the parameter, changing a time corresponding to the parameter, and changing a duration of a period during which the heater operates based on the profile.

16. The information processing device according to any one of claims 1 to 15, wherein
the inhaler device comprises a heater configured to heat the substrate to generate the aerosol, and
changing the profile comprises changing a duration of a period during which the heater performs preliminary heating.

17. An information processing method comprising:
generating a display image displaying a profile, the profile being information indicating a time-series change of a parameter related to an operation to be performed by an inhaler device that generates an aerosol by using a substrate, the operation being an operation of generating the aerosol; and
limiting a range within which the profile is changeable and changing the profile and display of the profile on the display image in accordance with a user operation on the profile on the generated display image, the user operation being a user operation for designating a change of the profile within the limited range within which the profile is changeable.

18. A program for causing a computer to execute:
generating a display image displaying a profile, the profile being information indicating a time-series change of a parameter related to an operation to be performed by an inhaler device that generates an aerosol by using a substrate, the operation being an operation of generating the aerosol; and
limiting a range within which the profile is changeable and changing the profile and display of the profile on the display image in accordance with a user operation on the profile on the generated display image, the user operation being a user operation for designating a change of the profile within the limited range within which the profile is changeable.
